# EUROPEAN PATENT APPLICATION

(11) **EP 4 729 537 A1**
(43) Date of publication of application: **22.04.2026**
(21) Application number: 24751298.1
(22) Date of filing: 13.06.2024
(51) Int. Cl.: C07K 16/12, A61P 31/04, C07K 14/35

(54) **B-LACTAMASE-INHIBITING ANTIBODY AND USE THEREOF IN THE TREATMENT AND/OR PREVENTION OF INFECTIONS CAUSED BY B-LACTAMASE-PRODUCING BACTERIA**

(30) Priority: 19.06.2023 ES 202330506
(71) Applicant: Universitat Politècnica de València, 46022 Valencia (ES)
(72) Inventor: PASCUAL-AHUIR GINER, María Desamparados, 46022 Valencia (ES); OSSET TRENOR, Paloma Pilar, 46011 Valencia (ES)
(74) Representative: Pons IP
(86) International application number: PCT/ES2024/070371
(87) International publication number: WO 2024/261361

(57) **Abstract**

In the present invention, this document refers to antibodies that inhibit β-lactamases, for example class A β-lactamase from *Mycobacterium abscessus,* as well as related compositions, uses thereof for and/or preventing infections caused by bacteria that produce β -lactamases.

## Description

The present invention belongs to the field of Biotechnology and Biomedicine and refers to a class A β-lactamases inhibitor antibody, and its use in the treatment of infections caused by β-lactamases-producing bacteria.

### BACKGROUND OF THE INVENTION

The global emergence of drug-resistant microorganisms is one of the most urgent health threats to be resolved. The overuse of antibiotics in healthcare, agriculture and livestock farming around the world has dramatically accelerated the emergence of nosocomial infections that mainly affect people with compromised immune systems. Although more than 1,200,000 people die from this cause each year, this number is expected to increase to more than 10 million in the coming years. Experts say that even common infections will no longer be treatable with conventional antibiotics soon.

The expression of β-lactamases enzymes is one of the resistance mechanisms of pathogenic bacteria against antibiotics. β-Lactamases are enzymes capable of degrading β-lactam antibiotics, therefore, these enzymes form an interesting target for the development of new therapeutic strategies against pathogenic bacteria (Bush, K. (2018). Antimicrobial agents and chemotherapy, 62 (10), e01076-18).

The development of antibodies capable of inhibiting β-lactamases is a promising alternative to treatment with current antibiotics, and based on this strategy there are different developments in the state of the art.

Document WO2020254861A1 describes the use of antibodies and antigen-binding fragments to neutralize β-lactamases and treat infections by antibiotic-resistant bacteria. The TEM-1 protein, a class A β-lactamase expressed by Gram-negative bacteria, was used as an antigen in obtaining antibodies for the treatment of bacterial infections.

In the publication "Conrath, KE, et al., (2001). Antimicrobial agents and chemotherapy, 45 (10), 2807-2812" describes the use of the proteins TEM-1 and Bcll, β-lactamases of type A and B respectively, to obtain single-domain antibodies inhibitors of β-lactamases. The β-lactamase TEM-1 is expressed in Gram-negative bacteria while the β-lactamase Bcll is expressed in Gram-positive bacteria.

In the publication "Cawez, F., et al, (2023). Antimicrobial Agents and Chemotherapy , 67 (4), e01499-22" discloses three soluble single-domain fragments derived from the unique variable region of camelid heavy chain (VHH) antibodies, which are inhibitors of the CMY-2 protein. , a class C β-lactamase produced by Gram-negative bacteria isolated from veterinary and human environments.

On the other hand, in the publication "Sohier, JS, et al., (2013). Biochemical Journal, 450 (3), 477-486" a single-domain antibody capable of inhibiting the class B β-Lactamase VIM-4, expressed in Gram-negative bacteria, was obtained.

However, the strategies described in the state of the art are limited to antibodies that inhibit class A, B and C β-lactamases from Gram-negative bacteria or class B β-lactamases from Gram-positive bacteria. Therefore, there is a need to generate new strategies that allow the inhibition of class A β-lactamase from Gram-positive bacteria.

### DESCRIPTION OF THE INVENTION

The present inventors have obtained antibodies capable of binding and inhibiting class A β-lactamase enzymes from Gram-positive bacteria, with an inhibition constant of less than 10 µM, which indicates an inhibition capacity at low concentrations (Table 2).

Thus, in a first aspect, the present invention relates to a class A β-lactamase inhibitor antibody from Gram-positive bacteria or a fragment thereof, hereinafter referred to as "antibody or fragment of the invention," characterized in that said antibody or fragment thereof inhibits a β-lactamase comprising an amino acid sequence with at least 80% identity to SEQ ID NO. 21, preferably at least 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO. 21.

In a preferred embodiment of the antibody or fragment of the invention, said antibody or fragment thereof comprises an inhibition constant (Ki) of less than 10 µM, more preferably comprises a Ki between 0.01 µM and 10 µM, between 0.01 µM and 8 µM, between 0.01 µM and 7 µM, between 0.01 µM and 6 µM, between 0.5 µM and 6 µM, or between 1 µM and 6 µM, against a β-lactamase comprising an amino acid sequence with at least 80% identity to SEQ ID NO. 21, preferably at least 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO. 21.

In the present invention, the polypeptide or β-lactamase comprising an amino acid sequence with at least 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO. 21 corresponds to a functionally equivalent variant of a polypeptide or β-lactamase comprising the amino acid sequence SEQ ID NO. 21.

In another even more preferred embodiment of the antibody or fragment of the invention, said fragment or antibody comprises a Ki of between 2.2 µM and 5.5 µM against a β-lactamase that comprises an amino acid sequence with at least 80% of identity with SEQ ID NO. 21, preferably at least 81%, 82%, 83%, 84%, 85%, 86%, 87%, 88%, 89%, 90%, 91%, 92%, 93 %, 94%, 95%, 96%, 97%, 98%, 99% or 100% identity with SEQ IDNO. 21.

The inhibition capacity of the antibody or fragment of the invention implies that said antibody or fragment is specific for β-lactamase that comprises an amino acid sequence with at least 80% identity with SEQ ID NO. 21, preferably at least 81 %, 82%, 83%, 84%, 85%, 86%, 87%, 88%, 89%, 90%, 91%, 92%, 93%, 94%, 95%, 96%, 97%, 98%, 99% or 100% identity with SEQ ID NO. 21.

The amino acid sequence SEQ ID NO. 21 comprises amino acids 31 to 263 of *M. abscessus* class A β-lactamase (NCBI reference *M. abscessus* class A β-lactamase: WP_005091054.1). The amino acid sequence SEQ ID NO. 21 corresponds to a fragment of the class A β-lactamase of *M. abscessus* that comprises the catalytic center without the 30 amino acids of the N-terminal end of said enzyme, making it a functional fragment.

Amino acid sequence of the class A β-lactamase fragment from *M. abscessus,* SEQ ID NO.21:

APDELASLEKDFGGRIGVYALDTGSGDTVGHRADERFLMCSTVKTFIVSAILRRRLSE
PGLLDQRIQYTQSDVLEWAPITSQHVSTGMTVSELCDATLRYSDNTGANLLITQLGGP

KETEKFVRSLGDNVTRMDRTEVQLNIPDGDLDTSTPQQLVANLRRLVLDEGLDSRGR
DLLTDWLKRNTTGDQSIRAAVPAGWTVADKTGGGFKGETNDIAVIWPPGRAPIVMAV
LTVPEDPTSTKGKPTIAAATRIVLRAFGA

The term "antibody", as used herein in the present invention, refers to a polypeptide or protein that comprises at least one "immunoglobulin variable domain" sequence, which refers to the structural unit of an immunoglobulin capable of bind to an antigen. As known to the person skilled in the art, an immunoglobulin variable domain consists of a polypeptide comprising 4 framework regions (FR ) referred to as "framework region 1" or "FR1" ; "framework region 2" or "FR2"; "framework region 3" or "FR3"; and "framework region 4" or "FR4", respectively; where said framework regions are interrupted by three "complementarity determining regions" or "CDR" called "complementarity determining region" or "CDR1" ; "complementarity determining region 2" or "CDR2"; and "complementarity determining region 3" or "CDR3," respectively. Thus, the general structure or sequence of an immunoglobulin variable domain can be indicated as follows: FR1 - CDR1 - FR2 - CDR2 - FR3 - CDR3 - FR4. CDRs are short sequences of amino acids, typically between 3 and 20 amino acids, which mediate the specific binding of the antibody to an antigen. Therefore, the immunoglobulin variable domain(s) confer specificity to an antibody for the antigen by carrying the antigen binding site. In the present invention, the term "immunoglobulin variable domain" may also be referred to as "antigen-binding variable region" or "antigen-binding domain."

The antibody of the present invention may be a complete antibody. Complete antibodies, for example from humans or mice, comprise at least two heavy polypeptide chains (H) linked by disulfide bonds, and in the case of antibodies of human origin they also comprise two light polypeptide chains (L), each of them linked to a heavy chain through disulfide bonds. Each heavy chain has a domain or variable region (VH) at one end followed by a plurality of constant regions. Each light chain has a domain or variable region (VL) at one end and a constant region at the other; the light chain constant region is opposite the first heavy chain constant region, and the light chain variable region is opposite the heavy chain variable region. Examples of complete antibodies include, without limitation, IgG or IgY antibodies.

In the present invention, the antibody may be an antibody composed of only two heavy chains where each chain comprises an immunoglobulin variable domain, said antibodies are called "heavy chain antibodies (HCAb)". HCAbs are produced naturally by camelids, where examples of camelids include without limitation camels, llamas, dromedaries or alpacas.

Thus, in a preferred embodiment, the antibody or fragment of the invention is human, mouse, rat, camelid, bird, a chimeric antibody or a humanized antibody.

The term "humanized antibody" refers to an antibody that has been designed to comprise one or more human framework regions in the variable region along with non-human (e.g., mouse or synthetic) complementarity determining regions (CDRs) of the heavy and/or light chain. In some embodiments, a humanized antibody comprises sequences that are fully human except for the CDR regions. Humanized antibodies are generally less immunogenic to humans than non-humanized antibodies, thus offering therapeutic advantages in certain situations.

The term "chimeric antibody" refers to an antibody that has been designed to comprise at least one human constant region.

In another preferred embodiment of the antibody or fragment of the invention, the antibody is selected from the list consisting of IgY, IgG1, IgG2, IgG3, IgG4 and a heavy chain antibody.

As is known to the person skilled in the art, antibodies can be monospecific or multispecific. In the present invention, the term "monospecific antibody" refers to an antibody capable of binding to a single antigen, while the term "multispecific antibody" refers to an antibody capable of binding to more than one antigen. Multispecific antibodies comprise at least two different immunoglobulin variable domains, each of which recognizes a different antigen (each domain is specific for a different epitope). The antibody of the present invention comprises at least one immunoglobulin variable domain that specifically binds to the polypeptide comprising the amino acid sequence with at least 80% identity to SEQ ID NO: 21, preferably at least 81%, 82 %, 83%, 84%, 85%, 86%, 87%, 88%, 89%, 90%, 91%, 92%, 93%, 94%, 95%, 96%, 97%, 98%, 99% or 100% identity with SEQ ID NO. 21.

Thus, in another preferred embodiment, the antibody or fragment of the invention is monospecific or multispecific; preferably where the multispecific antibody or fragment is bispecific.

In another preferred embodiment of the antibody or fragment of the invention, the antibody is monoclonal. The term "monoclonal antibody" refers to an antibody obtained from a population of substantially homogeneous antibodies, that is, the individual antibodies that make up the population are identical and/or bind to the same epitope, except for possible variant antibodies, for example. For example, they contain natural mutations or arise during the production of a monoclonal antibody preparation, said variants being generally present in minor quantities. Unlike polyclonal antibody preparations, which typically include different antibodies directed against different determinants (epitopes), each monoclonal antibody in a monoclonal antibody preparation is directed against a single determinant of an antigen.

The term "antibody fragment" as used herein refers to a functional portion of a complete antibody, wherein said functional portion preserves antigen-binding capacity. In the present invention, the antibody fragment comprises at least one immunoglobulin variable domain. Examples of antibody fragments include, but are not limited to, a single domain antibody (sdAb, either VL, VH or a VHH domain), IgG1 heavy chain antibody (HClgG1), IgG2 heavy chain antibody (HCIgG2), IgG3 heavy chain antibody (HCIgG3), IgG4 heavy chain antibody (HCIgG4), Fab, F(ab')2, Fv fragments, disulfide-linked Fv fragments (sdFv), an Fd fragment or scFv fragments.

Thus in a preferred embodiment of the antibody or fragment of the invention, the antibody fragment is selected from the list consisting of a single domain antibody (sdAb, either VL, VH or a VHH domain of camelid antibodies), an antibody IgG1 heavy chain antibody (HCIgG1), an IgG2 heavy chain antibody (HCIgG2), an IgG3 heavy chain antibody (HCIgG3), an IgG4 heavy chain antibody (HCIgG4), a Fab fragment, an F(ab')2 fragment, an Fv fragment, Fv fragments linked by a disulfide bridge (sdFv), an Fd fragment and a scFv fragment.

Antibody fragments have advantages over complete antibodies, since they are smaller in size, and therefore are more stable, are soluble and can more easily bind the epitope of the antigen to which they bind.

Thus, in a preferred embodiment of the antibody or fragment of the invention, the antibody is a single domain antibody (sdAb).

In the present invention, the terms "single domain antibody", "nanobody", "dAb" or "sdAb", used interchangeably, refer to a polypeptide comprising, preferably consisting of, a single immunoglobulin variable domain that binds specifically to an antigen. The single domain antibody may be a VH single domain antibody, a VL single domain antibody, or a VHH single domain antibody.

In the present invention, the terms "VHH single domain antibody", "VHH nanobodies", "VHH sdAb" or "VHH", used interchangeably, refer to a polypeptide consisting of a single camelid immunoglobulin variable domain or a polypeptide consisting of a single variable domain of humanized camelid immunoglobulin (Hamers-Casterman C, Atarhouch T, Muyldermans S, Robinson G, Hamers C, Songa EB, Bendahman N, Hamers R.: Naturally occurring antibodies devoid of light chains "; Nature 363, 446-448 (1993)).

The use of VHHs in the field of biomedicine, either alone or as part of a larger polypeptide, offers a number of significant advantages over the use of conventional VH and VL domains, scFvs or conventional antibody fragments (such as Fab- or F(ab')2 - fragments):
- A single domain is sufficient to bind an antigen with high affinity and selectivity, so there is no need to have two separate domains present, nor to ensure that these two domains are present in the correct conformation and spatial configuration (i.e. by using specially designed linkers, such as with scFvs);
- VHH domains can be expressed from a single gene and do not require folding or post-translational modifications;
- VHH domains can be easily designed into multivalent and multispecific (formed) formats;
- VHH domains are very soluble and do not tend to aggregate;
- VHH domains are very stable to heat, pH, proteases and other denaturing agents or conditions and, therefore, can be prepared, stored or transported without the need for refrigeration equipment, saving costs, time and environment;
- VHH domains are easy and relatively cheap to prepare, even at the scale necessary for production;
- VHH domains are relatively small (approximately 15 kDa, or 10 times smaller than a conventional IgG) compared to conventional 4-chain antibodies and their antigen-binding fragments, and therefore show high tissue penetration and they can be administered in higher doses than said conventional 4-chain antibodies and their antigen-binding fragments;
- VHH domains can display so-called cavity-binding properties (especially due to their extended CDR3 loop, compared to conventional VH domains) and can therefore also access targets and epitopes not accessible to 4-chain antibodies conventional and their antigen-binding fragments.

Camelid-derived VHH domains can be "humanized" by substituting one or more amino acid residues in the original VHH sequence with one or more amino acid residues found at the corresponding position(s) in a VH domain of a conventional four-chain human antibody (also referred to as "sequence optimization"). In addition to humanization, sequence optimization encompasses other sequence modifications through one or more mutations to provide enhanced VHH characteristics, such as the elimination of potential post-translational modification sites. A humanized VHH domain may contain one or more fully human framework region sequences.

Thus, in a more preferred embodiment, the antibody of the invention is a VHH single domain antibody or a humanized VHH single domain antibody.

In another preferred embodiment, the antibody or fragment of the invention is an isolated antibody or fragment, where the term "isolated" refers to a portion isolated from its natural environment, for example, a single antibody fragment that is substantially free from other antibodies, antibody fragments, and/or substantially free from cells or cellular materials.

According to the inventors' findings, the antibody of the invention is capable of acting as a β-lactamase inhibitor (Table 2). The term "β-lactamases" refers to those enzymes, expressed by Gram-positive or Gram-negative bacteria, capable of metabolizing and inactivating a β-lactam antibiotic. β-lactamases can inactivate β-lactam antibiotics by hydrolyzing the β-lactam ring of said antibiotics. β-lactamases are primarily classified into three classes depending on their activity: class A, B, and C. Class A β-lactamases comprise a serine residue in their catalytic center and exhibit penicillinase activity (examples of class A β-lactamases include, but are not limited to, enzymes from the SHV, CTX-M, or KPC subclasses). Class B β-lactamases are metallo-β-lactamases that require Zinc as a cofactor and exhibit cephalosporinase activity (examples of class B β-lactamases include, but are not limited to, enzymes from the VIM or Bcll subclasses). Class C β-lactamases comprise a serine residue in their catalytic center and exhibit cephalosporinase activity (examples of class C β-lactamases include, but are not limited to, enzymes from the CYM, ACT, DHA, FOX, or MIR subclasses) (Bush, K. (2018). Antimicrobial agents and chemotherapy, 62(10), e01076-18).

The term "inhibitor", and its grammatical derivations, refer in the present invention to the ability of an agent (e.g. the antibody of the invention) to block, partially block, interfere with, decrease, reduce or deactivate a biological molecule (e.g.. a β-lactamase enzyme), pathway or mechanism of action, thus in the present invention the term inhibitor refers to the ability of the antibody or fragment of the invention to inhibit a β-lactamase that comprises an amino acid sequence with at least one 80% identity with SEQ ID NO: 21, preferably at least 81%, 82%, 83%, 84%, 85%, 86%, 87%, 88%, 89%, 90%, 91%, 92% , 93%, 94%, 95%, 96%, 97%, 98%, 99% or 100%, preferably with an inhibition constant of less than 10 µM .

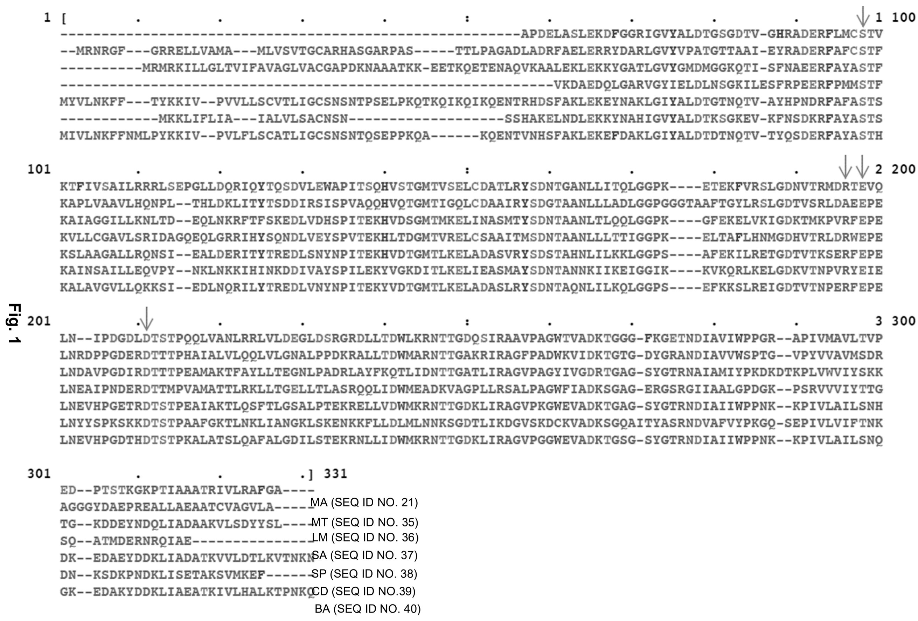

Surprisingly, the present inventors have obtained antibodies with inhibitory activity against β-lactamase, specifically the β-lactamase enzyme that comprises the sequence SEQ ID NO. 21. The enzyme comprising SEQ ID NO. 21 corresponds to a class A β-lactamase enzyme encoded by the Blamab gene of the bacterium *Mycobacterium abcessus* (NCBI reference of the *M. abscessus Blamab gene :* NG_088376.1; NCBI reference of the protein: WP_005091054.1). *M. abcessus* is a Gram-positive bacteria, commonly known by the deposit number ATCC19977, it can cause lung infections, skin infections, nervous system infections, bacteremias and eye infections. SEQ ID NO.21 preserves the amino acids of the catalytic site with respect to other Gram-positive class A β-lactamases sequences (Fig. 1).

The β-lactamase enzyme Blamab from Mycobacterium abscessus preserves the amino acids of the catalytic site along with other β-lactamase enzymes from other bacterial species, as indicated in Example 1 of the present invention. Therefore, the Blamab β-lactamase enzyme is a suitable target for the generation of inhibitory antibodies capable of acting against β-lactamases that share structural characteristics of the amino acids involved in their catalytic action. Based on the experiments provided by the present inventors, the antibodies of the invention were able to inhibit other β-lactamases from bacteria other than Mycobacterium abscessus (Example 4).

The term "identity," as used herein, refers to the proportion of identical amino acids between two peptides or proteins being compared. Sequence comparison methods are known in the state of the art, and include, but are not limited to, the BLASTP or BLASTN program, ClustalW and FASTA. We can consider that peptides or proteins with identity percentages of at least 80% will maintain the same properties as the β-lactamase that comprises the sequence SEQ ID NO. 21.

The antibodies obtained by the inventors presented a low inhibition constant (less than 10 µM ) against the polypeptide that comprises the amino acid sequence SEQ ID NO. 21, so they are capable of acting as β-lactamase inhibitors at very low concentrations.

In the present invention, the term "inhibition constant" or "Ki" is widely known to the person skilled in the art, which relates to the enzyme's ability to metabolize a substrate in the presence of an inhibitor (in the present invention, the inhibitor is the antibody of the invention). In the present invention, the term inhibition constant (Ki) has the meaning/interpretation of the person skilled in the art in view of common general knowledge. The inhibition constant can be calculated using methods known to those skilled in the art, including but not limited to, linearization methods such as the Lineweaver-Burk method, the Eadie-Hofstee method, the Hanes method, or the Scatchard method (Paolo Ascenzi, Maria Grazia Ascenzi, Gino Amiconi. Enzyme competitive inhibition, graphical determination of Ki and presentation of data in comparative studies, Biochemical Education, Volume 15, Issue 3, 1987, Pages 134-135, ISSN 0307-4412). The kinetic parameters of maximum velocity (Vmax) and the Michaelis constant (Km) can be obtained using methods known to the expert, such as, but not limited to, the nitrocefin assay (Kumar P, et al. Antimicrob Agents Chemother. 2017 Sep 22;61(10):e00866-17).

In the present invention, the inhibition constant (of the antibody or fragment of the invention) is obtained by the Lineweaver-Burk method, and where the kinetic parameters (maximum velocity, Vmax, and the Michaelis constant, Km, of the antibody or fragment of the invention) are obtained by means of a nitrocefin assay; preferably, the Vmax, Km and the inhibition constant of the antibody or fragment of the invention are obtained according to example 3 of the present invention.

In another preferred embodiment, the antibody or fragment of the invention comprises:
a) A CDR1 comprising, preferably consisting of, the amino acid sequence SEQ ID NO. 1: GTIFVYPY, a CDR2 comprising, preferably consisting of, the amino acid sequence SEQ ID NO. 2: EFVAGIGQGATTYY, and a CDR3 comprising, preferably consisting of, the amino acid sequence SEQ ID NO. 3: AYRYRDDVRRYGIDRYHT;
b) A CDR1 comprising, preferably consisting of, the amino acid sequence SEQ ID NO. 4: GTIFYYPY, a CDR2 comprising, preferably consisting of, the amino acid sequence SEQ ID NO. 5: EFVAGIDYGSTTYY, and a CDR3 comprising, preferably consisting of, the amino acid sequence SEQ ID NO. 6: VVYLVTRKGASQQDEIYS;
c) A CDR1 comprising, preferably consisting of, the amino acid sequence SEQ ID NO. 7: GTIFAQDY, a CDR2 comprising, preferably consisting of, the amino acid sequence SEQ ID NO. 8: EFVASIAYGTITYY, and a CDR3 comprising, preferably consisting of, the amino acid sequence SEQ ID NO. 9: VYPTPVTDYI;
d) A CDR1 comprising, preferably consisting of, the amino acid sequence SEQ ID NO. 10: GNISDLGT, a CDR2 comprising, preferably consisting of, the amino acid sequence SEQ ID NO. 11: EFVAGIGTGSNTYY, and a CDR3 comprising, preferably consisting of, the amino acid sequence SEQ ID NO. 12: VIAGRSYWIYFY; or
e) A CDR1 comprising, preferably consisting of, the amino acid sequence SEQ ID NO. 13: GTISAVSG, a CDR2 comprising, preferably consisting of, the amino acid sequence SEQ ID NO. 14: EFVASINRGSTTNY, and a CDR3 comprising, preferably consisting of, the amino acid sequence SEQ ID NO. 15: ALHGTGRVHV.

In another more preferred embodiment, the antibody or fragment of the invention comprises:
a) A CDR1 that comprises the amino acid sequence SEQ ID NO. 1, a CDR2 comprising the amino acid sequence SEQ ID NO. 2 and a CDR3 comprising the amino acid sequence SEQ ID NO. 3, where the antibody comprises a Ki of between 2.3 µM and 2.4 µM against β-lactamase that comprises an amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95% , 96%, 97%, 98%, 99% or 100% identity with SEQ ID NO. 21;
b) A CDR1 that comprises the amino acid sequence SEQ ID NO. 4, a CDR2 comprising the amino acid sequence SEQ ID NO. 5 and a CDR3 comprising the amino acid sequence SEQ ID NO. 6, where the antibody comprises a Ki of between 2.5 µM and 2.8 µM against β-lactamase that comprises an amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95% , 96%, 97%, 98%, 99% or 100% identity with SEQ ID NO. 21;
c) A CDR1 that comprises the amino acid sequence SEQ ID NO. 7, a CDR2 comprising the amino acid sequence SEQ ID NO. 8 and a CDR3 comprising the amino acid sequence SEQ ID NO. 9, where the antibody comprises a Ki of between 4.9 µM and 5.5 µM against β-lactamase that comprises an amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95% , 96%, 97%, 98%, 99% or 100% with SEQ ID NO. 21;
d) A CDR1 that comprises the amino acid sequence SEQ ID NO. 10, a CDR2 comprising the amino acid sequence SEQ ID NO. 11 and a CDR3 comprising the amino acid sequence SEQ ID NO. 12, where the antibody comprises a Ki of between 3.7 µM and 3.9 µM against β-lactamase that comprises an amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95 %, 96%, 97%, 98%, 99% or 100% with SEQ ID NO. 21; or
e) A CDR1 comprising the amino acid sequence SEQ ID NO. 13, a CDR2 comprising the amino acid sequence SEQ ID NO. 14 and a CDR3 comprising the amino acid sequence SEQ ID NO. 15, where the antibody comprises a Ki of between 4.4 µM and 5.4 µM against β-lactamase that comprises an amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95 %, 96%, 97%, 98%, 99% or 100% with SEQ ID NO. 21;
where the Ki (inhibition constant) is obtained by the Lineweaver-Burk method, and where the kinetic parameters (Vmax and Km) are obtained by a colorimetric assay with nitrocefin; preferably, the Vmax, Km and the inhibition constant are obtained according to example 3 of the present invention.

In another preferred embodiment of the antibody or fragment of the invention, the complementarity determining regions CDR1, CDR2 and CDR3 correspond to complementary regions of complementarity of the regions of the immunoglobulin variable domain of the heavy chain, which is why they are called CDRH1, CDRH2 and CDRH3 respectively.

In another even more preferred embodiment, the antibody of the invention comprises, preferably consists of, the amino acid sequence SEQ ID NO. 16, the amino acid sequence SEQ ID NO. 17, the amino acid sequence SEQ ID NO. 18, the amino acid sequence SEQ ID NO. 19 or the amino acid sequence SEQ ID NO. 20.

In the present invention, antibodies of sequence SEQ ID NO. 16, SEQ ID NO. 17, SEQ ID NO. 18, SEQ ID NO. 19 and SEQ ID NO. 20, are referenced as antibody B2, B3, B4, B5 and B6, respectively.

Amino acid sequence of the β-lactamase inhibitor B2 antibody, SEQ ID NO. 16:

MQVQLQESGGGLVQAGGSLRLSCAASGTIFVYPMGWYRQAPGKEREFVAGIGQG
ATTYYADSVKGRFTISRDNAKNTVYLQMNSLKPEDTAVYYCAAYRYRDDVRRYGIDR
YHTYWGQGTQVTVSS

The antibody of SEQ ID NO. 16 comprises CDR1, CDR2 and CDR3 of SEQ ID NO. 1, SEQ ID NO. 2 and SEQ ID NO.3, respectively.

Amino acid sequence of the β-lactamase inhibitor B3 antibody, SEQ ID NO. 17:

MQVQLQESGGGLVQAGGSLRLSCAASGTIFYYPYMGWYRQAPGKEREFVAGIDYGS
TTYYADSVKGRFTISRDNAKNTVYLQMNSLKPEDTAVYYCAVVYLVTRKGASQQDEIY
SYWGQGTQVTVSS

The antibody of SEQ ID NO. 17 comprises CDR1, CDR2 and CDR3 of SEQ ID NO. 4, SEQ ID NO. 5 and SEQ ID NO. 6, respectively.

Amino acid sequence of the β-lactamase inhibitor B4 antibody, SEQ ID NO. 18:

MQVQLQESGGGLVQAGGSLRLSCAASGTIFAQDYMGWYRQAPGKEREFVASIAYGT
ITYYADSVKGRFTISRDNAKNTVYLQMNSLKPEDTAVYYCAVYPTPVTDYIYWGQGTQ
VTVSS

The antibody of SEQ ID NO. 18 comprises CDR1, CDR2 and CDR3 of SEQ ID NO. 7, SEQ ID NO. 8 and SEQ ID NO. 9, respectively.

Amino acid sequence of the β-lactamase inhibitor B5 antibody, SEQ ID NO. 19:

MQVQLQESGGGLVQAGGSLRLSCAASGNISDLGTMGWYRQAPGKEREFVAGIGTG
SNTYYADSVKGRFTISRDNAKNTVYLQMNSLKPEDTAVYYCAVIAGRSYWIYFYYWG
QGTQVTVSS

The antibody of SEQ ID NO. 19 comprises CDR1, CDR2 and CDR3 of SEQ ID NO. 10, SEQ ID NO. 11 and SEQ ID NO. 12, respectively.

Amino acid sequence of the β-lactamase inhibitor B6 antibody, SEQ ID NO. 20:

MQVQLQESGGGLVQAGGSLRLSCAASGTISAVSGMGWYRQAPGKEREFVASINRGS
TTNYADSVKGRFTISRDNAKNTVYLQMNSLKPEDTAVYYCAALHGTGRVHVYWGQG
TQVTVSS

The antibody of SEQ ID NO. 20 comprises CDR1, CDR2 and CDR3 of SEQ ID NO. 13, SEQ ID NO. 14 and SEQ ID NO. 15, respectively.

The antibodies can covalently bind to molecules of interest such as therapeutic agents or detectable agents, thus forming conjugates that allow specific targeting of these molecules of interest to a target such as a cell expressing the antigen to which the antibody or fragment thereof binds.

Thus, in another preferred embodiment of the antibody or fragment of the invention, the antibody or fragment is linked to a therapeutic agent or a detectable marker.

In the present invention the term "therapeutic agent" refers to a compound or group of compounds that is administered to a subject for therapeutic, preventive medical or veterinary purposes.

In a more preferred embodiment of the antibody or fragment of the invention, the therapeutic agent is an antibacterial agent. The term "antibacterial agent" as used herein refers to a compound capable of inhibiting, reducing or preventing the growth of bacteria, capable of inhibiting or reducing the ability of a bacteria to produce infection in a subject, or capable of inhibiting or reducing the ability of a bacteria to multiply or remain infective in the environment. The term "antibacterial agent" also refers to compounds capable of decreasing the infectivity or virulence of bacteria. Examples of antibacterial agents include, without limitation, antimicrobial peptides, defensins, antibiotics, bacteriocins and endolysins.

In the present invention the terms "detectable marker" or "detectable agent", used interchangeably, refer to a compound or compounds capable of emitting a detectable signal which indicates the presence of a desired molecule, for example the antigen recognized by the antibody to which said marker or detectable agent is attached. Examples of a detectable label include, without limitation, a fluorescent compound, a bioluminescent compound, a chemiluminescent compound, a radioisotope, a prosthetic group, a positron-emitting metal, and a non-radioactive paramagnetic metal ion. The examples described in this paragraph are within the scope of the present invention.

The detectable marker signal can be determined by any of the methods known to the person skilled in the art, which include, without limitation, fluorescence spectroscopy techniques, computed tomography (CT), magnetic resonance (MR), optical imaging, tomography. single photon emission computed tomography (SPECT), positron emission tomography (PET), x-ray imaging, gamma ray imaging or similar.

The therapeutic agent or detectable marker is linked to the antibody or fragment of the invention by covalent bonds, preferably by the functional groups (e.g. -OH, -NH₂, -SH) of the side chains of the amino acids of said antibody or fragment.

In another preferred embodiment, the antibody or fragment of the invention is immobilized on a solid support, a lipid particle, a nanoparticle, on the surface of a virus or on the surface of a cell.

As used herein, the term "solid support" is used in reference to any solid or stationary material to which reagents such as antibodies, fragments thereof, or antigens are attached. For example, solid supports include plastic, but are not limited to, glasses, crystal, microtiter plate wells, microscope slides, coverslips, lateral flow assay strips, cell culture flasks or gels.

**In** the present invention the term "lipid particle" refers to a particle composed mainly of lipids, for example, liposomes or micelles.

**In** the present invention the term "nanoparticle" means a particle that has a size within the nanometer scale, such as, for example, metallic nanoparticles of gold, silver or copper, silica nanoparticles or polymeric nanoparticles.

The antibody or fragment of the invention may be immobilized attached to proteins or lipids on the surface of a virus or cell. The cell on which the antibody or fragment of the invention can be immobilized can be either a prokaryotic cell (e.g. *Escherichia coli*) or a eukaryotic cell (e.g. *Saccharomyces cerevisiae* or a non-human animal cell or a human cell). Thus, the antibody or fragment of the invention can be attached to or form part of a display system in yeast, phages or bacteria; or in animal or human cells such as CART cells.

The antibody or fragment of the invention may be immobilized by, without limitation, electrostatic interactions, covalent binding (e.g., peptide bond or avidin-biotin binding), adsorption, or similar method.

**In** another aspect, the present invention relates to a pharmaceutical composition, hereinafter the "pharmaceutical composition of the invention", which comprises the antibody or fragment of the invention.

In the present invention, the term "pharmaceutical composition" refers to any substance used for diagnosis, prevention, alleviation, treatment or cure of a disease in humans or animals. The pharmaceutical composition of the invention can be used both alone and in combination with other pharmaceutical compositions. In a preferred embodiment, the pharmaceutical composition further comprises a pharmaceutically acceptable carrier or excipient.

In a preferred embodiment, the pharmaceutical composition of the invention further comprises a pharmaceutically acceptable excipient and/or carrier.

The term "excipient" refers to a substance that helps the absorption of the pharmaceutical composition, comprising the antibody of the invention, stabilizes it or helps in its preparation in the sense of giving it a consistency, shape, flavor or any other specific functional characteristic. Thus, the excipients could have the function of holding the ingredients together such as starches, sugars or cellulose, sweetening function, coloring function, protective function such as isolating it from air and/or humidity, filling function of a pill, capsule or any other form of presentation such as dibasic calcium phosphate, disintegrating function to facilitate the dissolution of the components and their absorption, without excluding other types of excipients not mentioned in this paragraph.

A "vehicle" refers to those substances, or combination of substances, known in the pharmaceutical sector, used in the preparation of pharmaceutical forms of administration and includes, but is not limited to, solids, liquids, solvents or surfactants. The vehicle can be an inert substance or an action analogous to any of the compounds of the present invention and whose function is to facilitate the incorporation of the drug, as well as other compounds, allow better dosage and administration or give consistency and shape to the pharmaceutical composition. When the presentation form is liquid, the vehicle is the diluent.

The term "pharmacologically acceptable" (or "pharmacologically acceptable") refers to the compound being permitted and evaluated so that it does not cause harm to the organisms to which it is administered.

The pharmaceutical composition of this invention can be provided by any route of administration, for which said composition will be formulated in the pharmaceutical form appropriate to the chosen route of administration. In a preferred embodiment, the pharmaceutical composition of the invention can be formulated in solid, semi-solid, liquid or gaseous forms, such as tablet, capsule, powder, granule, ointment, solution, suppository, injection, inhalant, gel, cream, microsphere or aerosol. According to an even more preferred embodiment of the present invention, the pharmaceutical composition is presented in a form adapted for oral, intravenous, intramuscular, intramuscular, intra-arterial, intravenous, vaginal, intradermal, subcutaneous, topical, ophthalmic or inhalation administration.

The orally administrable form refers to a physical state that enables oral administration. Such orally administrable form is selected from the list comprising, but not limited to, drops, syrup, herbal tea, elixir, suspension, extemporaneous suspension, drinkable vial, tablet, capsule, granule, lozenge, pill, tablet, troche, or lyophilized form.

The form adapted to topical administration refers to a physical state that can allow its topical administration. Said form adapted to topical administration is selected from the list comprising, but not limited to, paste, cream, gel or ointment.

The form adapted to parenteral administration refers to a physical state that can allow its injectable administration, that is, preferably in a liquid state. Parenteral administration can be carried out by intramuscular, intra-arterial, intravenous, ophthalmic, intradermal or subcutaneous administration routes, but not limited only to these types of parenteral administration routes.

The form adapted to administration by inhalation refers to a physical state that can allow its administration by nasal or buccal inhalation, preferably in the form of powder, nebulized or aerosol.

Another possibility is that the pharmaceutical composition is presented in a form adapted for sublingual, intrathecal, bronchial, lymphatic, rectal or transdermal administration.

In another aspect, the present invention relates to the antibody, fragment or pharmaceutical composition of the invention for use as a medicament.

The term "medicine" refers to any substance or combination of substances that is presented as having properties for the treatment or prevention of diseases in a subject, or that can be used in a subject or administered to a subject for the purpose of restoring, correct or modify physiological functions by exercising a pharmacological, immunological or metabolic action, or establishing a medical diagnosis.

In another aspect, the present invention relates to the antibody, fragment or pharmaceutical composition of the invention for use in the treatment and/or prevention of an infection caused by a β-lactamase-producing bacteria in a subject, hereinafter forward "antibacterial use of the invention".

In the present invention, the term "β-lactamase-producing bacteria" refers to a bacteria that expresses β-lactamase and is therefore capable of metabolizing β-lactam antibiotics, which is why it presents resistance to said antibiotics. As known to the person skilled in the art, examples of bacteria that produce β-lactamase include, without limitation, pathogenic bacteria belonging to the genera *Mycobacterium , Pseudomona, Klebsiella, Listeria, Staphylococcus, Enterococcus, Streptococcus, Clostridium, Bacillus or Corynebacterium.*

In the present invention, the term "treatment" refers to combating the effects caused as a consequence of a disease or pathological condition of interest in a subject (preferably mammal, and more preferably a human) that includes:
(i) inhibit the disease or pathological condition, that is, stop its development;
(ii) alleviate the disease or pathological condition, that is, cause the regression of the disease or pathological condition or its symptomatology;
(iii) stabilize the disease or pathological condition.

In the present invention, the term "prevention" refers to preventing the occurrence of the disease, that is, preventing the disease or pathological condition from occurring in a subject (preferably mammal, and more preferably a human), in particular, when said subject has a predisposition for the pathological condition.

In a preferred embodiment of the antimicrobial use of the invention, the antibody, fragment or pharmaceutical composition of the invention is administered to the subject in a therapeutically effective amount.

n the present invention, the expressions "therapeutically effective dose" or "therapeutically effective amount," used interchangeably herein, refer to the quantity or dose of the antibody, fragment, or pharmaceutical composition of the invention that, when administered to a subject, preferably a mammal, and more preferably a human, is sufficient to produce the treatment and/or prevention of an infection caused by a β-lactamase-producing bacterium in the subject. The therapeutically effective amount will vary, for example, according to the metabolic stability and duration of action of the extracts; the age, body weight, general health condition, sex, and diet of the subject; the mode and timing of administration; the rate of excretion, combination with other drugs; the severity of the disease or the particular pathological condition of the subject. It is routine practice for a person skilled in the art, for example, a medical specialist, to determine the therapeutically effective dose for each subject, preferably a human, based on their own knowledge and the physiological conditions or variables of the subject.

The term "subject", as understood in the present invention, refers to a human or a non-human animal, where examples of non-human animal include, without limitation, dogs, cats, birds, cows, pigs, goats, sheep, fish, reptiles, non-human primates, mice or rats. Thus, in a preferred embodiment of the antimicrobial use of the invention, the subject is selected from a human or a non-human animal.

In a more preferred embodiment of the antimicrobial use of the invention, where the bacteria is a bacteria that produces a class A β-lactamase.

In another preferred embodiment of the antimicrobial use of the invention, the bacteria is Gram positive.

In an even more preferred embodiment of the antimicrobial use of the invention, the bacteria is selected from the list consisting of *Mycobacterium abscessus, Pseudomonas aeruginosa, Mycobacterium tuberculosis, Mycobacterium leprae, Klebsiella pneumoniae, Listeria monocytogenes, Staphylococcus aureus, Enterococcus faecium, Enterococcus faecalis Streptococcus pneumoniae, Streptococcus agalactiae , Streptococcus pyogenes, Clostridium botulinum, Clostridium novyi, Clostridium septicum, Clostridium perfringens, Clostridium tetani, Clostridium difficile, Clostridium mangenotii, Bacillus anthracis, and Corynebacterium diphtheriae.*

In a preferred embodiment of the antimicrobial use of the invention, the antibody, fragment or pharmaceutical composition of the invention is administered to the subject by a route of administration selected from the list consisting of oral, intravenous, intramuscular, intramuscular, intra-arterial, intravenous, intradermal, vaginal, subcutaneous, topical, ophthalmic and by inhalation.

In an even more preferred embodiment of the antimicrobial use of the invention, the infection is selected from the list consisting of lung infection, urinary tract infection, skin infection, nervous system infection, bacteremia, septicemia, ocular infection, and sexually transmitted.

In another aspect, the present invention relates to a polynucleotide encoding the antibody or fragment of the invention, hereinafter "the polynucleotide of the invention."

The terms "nucleic acid" or "polynucleotide," used interchangeably, refer to a polymer of nucleotides of any length, including DNA and RNA. The nucleotides can be deoxyribonucleotides, ribonucleotides, modified nucleotides or any substrate that can be incorporated into a DNA or RNA polymer by a polymerase or by a synthetic reaction.

The term "encodes" refers to the genetic code that determines how a nucleotide sequence is translated into a polypeptide or protein. The order of nucleotides in a sequence determines the order of amino acids throughout a polypeptide or protein.

In another aspect, the present invention relates to an expression vector, hereinafter "the expression vector of the invention" comprising the polynucleotide of the invention.

A vector is a nucleic acid molecule used to transfer genetic material to a cell. Apart from such genetic material, a vector can also contain different functional elements that include transcription control elements, such as promoters or operators, transcription factor binding regions or enhancers, and control elements for initiating and terminating translation. Vectors include, but are not limited to: plasmids, cosmids, viruses, phages, recombinant expression cassettes and transposons. Some vectors are capable of replicating or dividing autonomously once they are introduced into the host cell, such as bacterial vectors with a bacterial origin of replication or mammalian episomal vectors. Other vectors can integrate into the genome of the host cell and thus replicate along with the cellular genome. An expression vector is one capable of directing the expression of genes to which it has been operatively linked. An expression vector is used for the translation and transcription of a gene of interest, usually controlled by a promoter. A promoter is a sequence of nucleotides that controls the translation of the gene of interest. The promoter is operably linked to the gene of interest. "Operably linked" refers to the functional relationship and location of the promoter sequence with respect to the gene of interest, for example, a promoter or enhancer is operably linked to a coding sequence if it affects transcription of the sequence. In general, an operatively linked promoter is contiguous to the sequence of interest. However, an enhancer does not have to be contiguous to the sequence of interest to control its expression.

In another aspect, the present invention relates to a host cell comprising the polynucleotide of the invention or the expression vector of the invention. The cell may be prokaryotic, for example, but without limitation, the cell may be a bacteria such as *E*. *coli* that is used to produce the antibody or fragment of the invention. The cell may be eukaryotic, such as, but not limited to, a yeast or insect cell, which is used to produce the antibody or fragment of the invention.

In another aspect, the present invention relates to a polypeptide consisting of the amino acid sequence SEQ ID NO. 21.

In another aspect, the present invention relates to the *in vitro use* of a polypeptide comprising an amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95% , 96%, 97%, 98%, 99% or 100% identity with SEQ ID NO. 21, for obtaining antibodies or fragments thereof, from now on "use for obtaining the invention". Preferably, the production use of the invention is aimed at obtaining antibodies, or fragments thereof, which specifically bind to β-lactamases.

In the method for obtaining the invention, the polypeptide comprising an amino acid sequence with at least 80% identity to SEQ ID NO. 21, preferably at least 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO. 21, is used as an antigen for obtaining antibodies or fragments thereof.

In the present invention, the term "antigen" refers to a molecule comprising an epitope to which an antibody, or fragment thereof, is specifically bound by at least one immunoglobulin variable domain. So, in the use of obtaining the invention, said antigen allows selecting antibodies, or fragments thereof, specific for β-lactamases, preferably Gram-positive class A β-lactamases.

According to the common knowledge of the person skilled in the art, molecules of peptide nature, such as the polypeptide comprising the amino acid sequence SEQ ID NO. 21, can be used as antigens to obtain specific antibodies by *in vitro methods* that include, without limitation, the protein display systems in viruses ( *phage display* or *phage surface display* ), protein display system in prokaryotes (*bacterial display* or *bacterial surface display* ), for example *E.coli* , or protein display systems in eukaryotes, for example in yeast (*yeast display* or *yeast surface display* ) (Valldorf, Bernhard, et al., Biological Chemistry , vol. 403, no. 5-6, 2022, pp. 455-477).

In a preferred embodiment of the use of obtaining the invention, the antibodies or fragments thereof are human, mouse, rat, camelid, bird, chimeric antibodies or humanized antibodies.

In another preferred embodiment of the production use of the invention, the antibodies are selected from the list consisting of IgY, IgG1, IgG2, IgG3 IgG4 and heavy chain antibodies (HCAb).

In another preferred embodiment of the production use of the invention, the antibodies are monoclonal.

In another preferred embodiment of the production use of the invention, the antibody fragments are selected from the list consisting of single domain antibodies (sdAb, either VL, VH or a VHH domain of camelid antibodies), s-chain antibodies IgG1 heavy chain antibodies (HCIgG1), IgG2 heavy chain antibodies (HCIgG2), IgG3 heavy chain antibodies (HCIgG3), IgG4 heavy chain antibodies (HCIgG4), Fab fragments, F(ab')2 fragments, Fv fragments, bound Fv fragments by a disulfide bridge (sdFv), Fd fragments and scFv fragments.

In a preferred embodiment of the production use of the invention, the antibodies are single domain antibodies.

In a more preferred embodiment of the production use of the invention, the antibodies are VHH single domain antibodies or humanized VHH single domain antibodies.

In another aspect, the present invention refers to an *in vitro* method for obtaining antibodies or antibody fragments against β-lactamase, hereinafter "method for obtaining the invention", where said method comprises:
a) providing a library of nucleic acids that encode antibodies or fragments thereof, wherein said antibodies or fragments comprise at least one immunoglobulin variable domain;
b) introducing the library from step a) into a surface display system selected from the list consisting of a phage surface display system, a prokaryotic surface display system and a eukaryotic surface display system;
c) contacting the exposure system of step b) with the antigen consisting of a polypeptide comprising an amino acid sequence with at least 80% identity with SEQ ID NO: 21; and
d) select antibodies, or fragments thereof, that specifically bind to the antigen from step b).

The expression "antibodies or antibody fragments against β-lactamase" in the method of obtaining the invention refers to the fact that the antibodies or fragments thereof are specific for β-lactamase, preferably a β-lactamase that comprises a amino acid sequence with at least 80% identity with SEQ ID NO. 21, preferably at least 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO. 21.

In the present invention, the term "nucleic acid library" refers to a collection of nucleic acids or polynucleotides, preferably deoxyribonucleic acid (DNA), where the DNA can be genomic DNA or complementary DNA (cDNA, synthetic DNA transcribed from a specific mRNA using the enzyme reverse transcriptase). As known by those skilled in the art, the nucleic acid library for the in vitro production of antibodies or fragments thereof can originate from a cell source such as immunized B cells, non-immunized B cells, or it can be synthetically generated. In the case of immunized B cell sources, these cells can come from a subject (e.g., a human or a non-human mammal, preferably a camelid) that has been immunized with an antigen of interest (e.g., a polypeptide comprising the amino acid sequence SEQ ID NO. 21) for which the antibodies or fragments thereof are to be obtained that specifically bind to that antigen. Once the B cells from the immunized subject have been isolated, nucleic acids are extracted and amplified using specific primers for the genomic regions encoding the antibodies or fragments thereof (e.g., the genome sequences encoding the variable regions of the antibodies). On the other hand, nucleic acid libraries for the in vitro production of antibodies or fragments thereof can be obtained from B cells from a non-immunized subject ("naive" libraries). In this case, nucleic acids are extracted and amplified using specific primers for the genomic regions encoding the antibodies or fragments thereof, and variability is introduced into the CDR regions during amplification through directed mutation techniques. Additionally, nucleic acid libraries for the in vitro production of antibodies or fragments thereof can be synthetically generated, for example, by assembling known and synthetic FR sequences, where the CDRs are varied using directed mutation techniques. The directed mutation methodologies that can be used in the optimization of libraries for the production of antibodies or fragments thereof include, without limitation, CRISPR-Cas or error-prone polymerase chain reaction (error-prone PCR) (Valldorf, Bernhard, et al., Biological Chemistry, vol. 403, no. 5-6, 2022, pp. 455-477).

Thus, in a preferred embodiment of the method of the invention, the library of step a) is obtained from B cells isolated from a non-immunized subject, by assembly of synthetic FR sequences with CDRs mutated in a directed manner, or from B cells. of a subject immunized with polypeptide comprising an amino acid sequence with at least 80% identity with SEQ ID NO: 21, preferably at least 81%, 82%, 83%, 84%, 85%, 86%, 87 %, 88%, 89%, 90%, 91%, 92%, 93%, 94%, 95%, 96%, 97%, 98%, 99% or 100%; where the immunized or non-immunized subject is selected from a human or a non-human mammal, preferably where the selected one consists of a camelid, a mouse or a rat.

Following the provision of the library from step a) of the method of the invention, it is introduced into a display system selected from the list consisting of a phage display system, a prokaryotic display system, and a eukaryotic display system, in accordance with step b).

In the present invention, the terms "exposure system" or "surface exposure system", used interchangeably, refer to viruses or cells that express polypeptides of interest on their surface, for this the nucleic acid that encodes the polypeptide of interest is introduced into the surface exposure system, which comprises translating said nucleic acid. The nucleic acid encoding the polypeptide of interest can be introduced into it as part of a vector such as a plasmid. The introduction of the nucleic acid molecules from the library of step b) is carried out by known techniques such as, without limitation, electroporation, calcium phosphate exposure or viral transfection in the case of cellular display systems. Thus, the exposure system after step b) of the method of the present invention, expresses and presents on its surface the antibodies or fragments thereof encoded by the nucleic acids of the library in step a).

In a preferred embodiment of the method of obtaining the invention, the eukaryotic surface display system is a mammalian cell surface display system or a yeast surface display system (*yeast display, yeast surface display* or *YSD*).

Following step b) of the method of the invention, the display system is contacted with the antigen in step c), and the antibodies or fragments thereof (expressed on the surface of the display system) that specifically bind to said antigen are selected.

In aspect c) of the method of obtaining the invention, the antigen can be attached to a solid support, such as, but not limited to, a gel, glass, plastic or gold nanoparticles.

Once the exposure system has been contacted with the antigen in step c), selection step d) is carried out, preferably by a washing operation under conditions in which the binding between the antibodies or antigen fragments is maintained. the same exposed on the surface of the exposure system, and the antigen, while the weaker bonds are eliminated. Methods for selecting antibodies that specifically bind to an antigen are known to the person skilled in the art, and include, without limitation, immunological techniques such as MACS (magnetic *activated cell sorting* ) fluorescent activated cell sorting) or ELISA (enzyme linked immunosorbent assay).

In another preferred embodiment of the method of obtaining the invention, steps c) and d) are repeated at least once.

Following the selection in step d), nucleic acids comprised in the phages, prokaryotic cells, or eukaryotic cells of the display system are isolated. After isolation, the sequence analysis of said nucleic acids is performed, thereby determining the nucleic acid sequence or sequences that encode for the antibodies or fragments thereof capable of specifically binding to the antigen. Sequence analysis can be carried out using known methods such as, but not limited to, Sanger sequencing or next-generation sequencing. Subsequently, the nucleic acids encoding the selected antibodies or fragments thereof obtained by the method of the invention are cloned and expressed in a host cell.

In a preferred embodiment of the method of obtaining the invention, the antibodies or fragments thereof are human, mouse, rat, camelid, bird, chimeric antibodies or humanized antibodies.

In another preferred embodiment of the method of obtaining the invention, the antibodies are selected from the list consisting of IgY, IgG1, IgG2, IgG3, and heavy chain antibodies (HCAb).

In another preferred embodiment of the method of obtaining the invention, the antibodies are monoclonal.

In another preferred embodiment of the method of obtaining the invention, the antibody fragments are selected from the list consisting of single domain antibodies (sdAb, either VL, VH or a VHH domain of camelid antibodies), heavy chain antibodies IgG1 (HCIgG1), IgG2 heavy chain antibodies (HCIgG2), IgG3 heavy chain antibodies (HCIgG3), IgG4 heavy chain antibodies (HCIgG4), Fab fragments, F(ab')2 fragments, Fv fragments, Fv fragments linked by a disulfide bridge (sdFv), Fd fragments and scFv fragments.

In a preferred embodiment of the method of obtaining the invention, the antibodies are single domain antibodies.

In a more preferred embodiment of the method of obtaining the invention, the antibodies are VHH single domain antibodies or humanized VHH single domain antibodies.

In another preferred embodiment of the method of obtaining the invention, in step c) of the invention a polypeptide comprising an amino acid sequence with at least 81%, preferably at least 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO. 21.

The terms "antibody", "immunoglobulin variable domain", "heavy chain antibody (HCAb)", "humanized antibody", "chimeric antibody", "antibody fragment", "single domain antibody", "β-lactamase" and "inhibitor" have been defined and explained in previous paragraphs in the antibody and fragment aspect of the invention, and both said definitions and their preferred embodiments are applicable to the use and method aspects of the invention.

In the present invention, the ranges of values such as the inhibition constant refer not only to the values within said range, but also include the values of the upper and lower limits of said ranges.

### DESCRIPTION OF THE FIGURES

**Fig.1****.** Multiple alignment of different Gram-positive class a β-lactamases. Arrows indicate residues that are especially important in the structural and functional characteristics of the enzyme; S70 and E166 of the catalytic site, and residues R164 and D179 contained in the Ω loop. MA: amino acid sequence of the antigenic protein of *Mycobacterium abscessus* SEQ ID NO. 21. MT: amino acid sequence of class A β-Lactamase from *Mycobacterium tuberculosis,* SEQ ID NO. 35 (NCBI Reference Sequence: WP_057351649.1). LM: amino acid sequence of β-Lactamase class A *Listeria monocytogenes,* SEQ ID NO. 36 (NCBI Reference Sequence: WP_036094553.1). SA: amino acid sequence of β-Lactamase class A from *Staphylococcus aureus,* SEQ ID NO. 37 (NCBI Reference Sequence: WP_149508696.1). SP: class A β-Lactamase amino acid sequence of *Streptococcus pneumoniae,* SEQ ID NO. 38 (GenBank: CGG65551.1). CD: amino acid sequence of Clostridium Difficile class A β-Lactamase, SEQ ID NO. 39 (GenBank: HBH2479975.1). BA: amino acid sequence of β-Lactamase class A from *Bacillus anthracis,* SEQ ID NO. 40 (GenBank: PES83414.1).

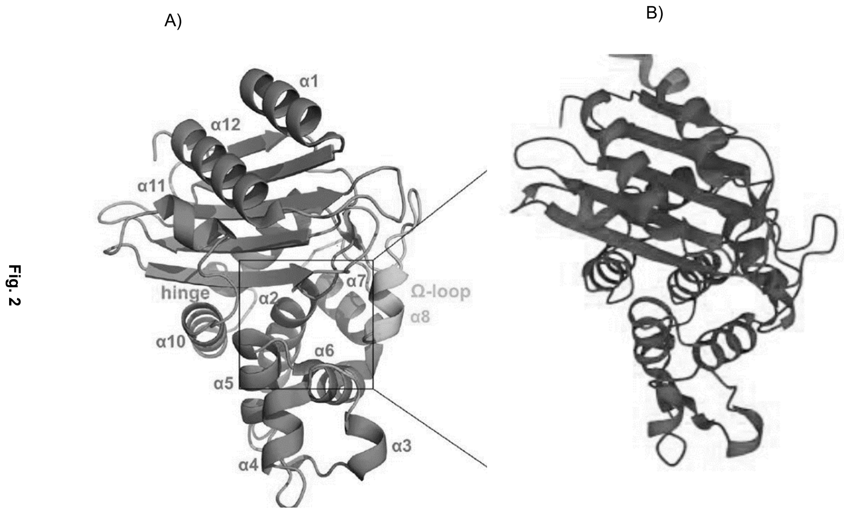

**Fig. 2****.** Comparison of the class A β-lactamase of *M.abscessus* encoded by the Blamab gene, with the KPC-2 protein of *Klebsiella Pneumoniae* . A) solved structure resulting from the crystallization of KPC-2 (SEQ ID NO. 32) B) *M.abscessus* class A β-lactamase model generated by the Alphafold artificial intelligence tool.

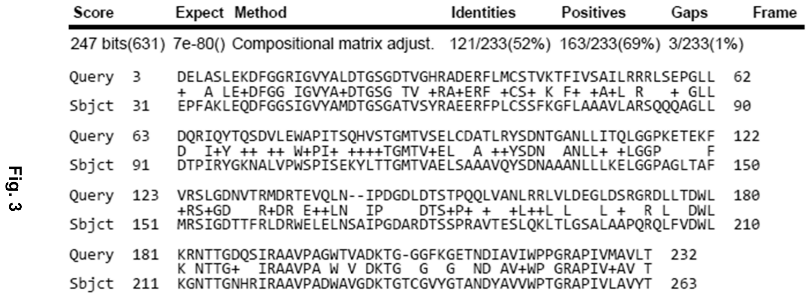

**Fig. 3****.** Alignment of the amino acid sequence of the class A β-lactamase of *M.abscessus* (SEQ ID NO. 21 ) and the β-lactamase *KPC-2 from Pseudomonas aeruginosa* (SEQ ID NO. 32) .

### EXAMPLES

Next, the invention will be illustrated through tests carried out by the inventors, which demonstrate the effectiveness of the product of the invention.

### Example 1. Design and process for obtaining class A B-lactamase.

The antigen used to obtain antibodies against β-lactamase is a fragment of the protein encoded by the Blamab gene of *Mycobacterium abscessus,* SEQ ID NO. 21 (NCBI reference of the Blamab gene of *M*. *abscessus:* NG_088376.1; NCBI reference of the protein encoded by the Blamab gene of *M*. *abscessus:* WP_005091054.1). The protein encoded by the Blamab gene of *Mycobacterium abscessus* has class A β-lactamase activity and consists of the amino acid sequence SEQ ID NO. 31 (NCBI reference of the protein encoded by the Blamab gene of *M*. *abscessus:* WP_005091054.1).

Complete amino acid sequence of β-lactamase from *Mycobacterium abscessus,* SEQ ID NO. 31:

MISRRALLVGGVSAVGVVAAGCSRNGNRRPAPDELASLEKDFGGRIGVYALDTGSGD
TVGHRADERFLMCSTVKTFIVSAILRRRLSEPGLLDQRIQYTQSDVLEWAPITSQHVST
GMTVSELCDATLRYSDNTGANLLITQLGGPKETEKFVRSLGDNVTRMDRTEVQLNIPD
GDLDTSTPQQLVANLRRLVLDEGLDSRGRDLLTDWLKRNTTGDQSIRAAVPAGWTVA
DKTGGGFKGETNDIAVIWPPGRAPIVMAVLTVPEDPTSTKGKPTIAAAT RIVLRAFGA

Amino acids 1 to 30 of the amino acid sequence SEQ ID NO. 31 correspond to the sequence of which it is processed and eliminated immediately in the periplasm and is not part of the protein released into the periplasmic space.

Class A β-lactamases are a very large family of proteins in Gram-positive bacteria. The antigenic protein was chosen based on its high homology with other Gram-positive β-lactamases that exist and are associated with severe infectious diseases, where this homology was determined by a multiple alignment of several Gram-positive β-lactamases against the peptide of SEQ ID NO. 21. The alignment was carried out with the NCBI BLASTp tool, and the amino acid sequences SEQ ID NO.21, SEQ ID NO. 35, SEQ ID NO. 36, SEQ ID NO. 37, SEQ ID NO. 38, SEQ ID NO. 39 and SEQ ID NO. 40. Great homology is demonstrated in the amino acids of the catalytic site between the sequence SEQ ID NO. 21 with the rest of the amino acid sequences of Gram-positive class A β-lactamases, and their essential residues S70 and E166, in addition to R164 and D179 contained in the Ω loop and characteristic of this family (Fig. 1).
- *Mycobacterium abscessus* antigenic protein SEQ ID NO. 21.
- Amino acid sequence of class A β-Lactamase from *Mycobacterium tuberculosis,* SEQ ID NO. 35 (NCBI Reference Sequence: WP_057351649.1): 
MRNRGFGRRELLVAMAMLVSVTGCARHASGARPASTTLPAGADLADRFAEL
ERRYDARLGVYVPATGTTAAIEYRADERFAFCSTFKAPLVAAVLHQNPLTHLD
KLITYTSDDIRSISPVAQQHVQTGMTIGQLCDAAIRYSDGTAANLLLADLGGPG
GGTAAFTGYLRSLGDTVSRLDAEEPELNRDPPGDERDTTTPHAIALVLQQLVL
GNALPPDKRALLTDWMARNTTGAKRIRAGFPADWKVIDKTGTGDYGRANDIA
VVWSPTGVPYVVAVMSDRAGGGYDAEPREALLAEAATCVAGVLA
- Amino acid sequence of β-Lactamase class A *Listeria monocytogenes* SEQ ID NO. 36 (NCBI Reference Sequence: WP_036094553.1): 
MRMRKILLGLTVIFAVAGLVACGAPDKNAAATKKEETKQETENAQVKAALEKL
EKKYGATLGVYGMDMGGKQTISFNAEERFAYASTFKAIAGGILLKNLTDEQLN
KRFTFSKEDLVDHSPITEKHVDSGMTMKELINASMTYSDNTAANLTLQQLGGP
KGFEKELVKIGDKTMKPVRFEPELNDAVPGDIRDTTTPEAMAKTFAYLLTEGN
LPADRLAYFKQTLIDNTTGATLIRAGVPAGYIVGDRTGAGGSYGTRNAIAMIYP
KDKDTKPLVWVIYSKKTGKDDEYNDQLIADAAKVLSDYYSL
- Amino acid sequence of β-Lactamase class A from *Staphylococcus aureus* SEQ ID NO: 37 (NCBI Reference Sequence: WP_149508696.1): 
VKDAEDQLGARVGYIELDLNSGKILESFRPEERFPMMSTFKVLLCGAVLSRIDA
GQEQLGRRIHYSQNDLVEYSPVTEKHLTDGMTVRELCSAAITMSDNTAANLLL
TTIGGPKELTAFLHNMGDHVTRLDRWEPELNEAIPNDERDTTMPVAMATTLRK
LLTGELLTLASRQQLIDWMEADKVAGPLLRSALPAGWFIADKSGAGERGSRGI
IA ALGPDGKPSRVVVIYTTGSQATMDERNRQIAE
- Amino acid sequence of β-Lactamase class A from *Streptococcus pneumoniae* SEQ ID NO. 38 (GenBank: CGG65551.1): 
MYVLNKFFTYKKIVPVVLLSCVTLIGCSNSNTPSELPKQTKQIKQIKQENTRHD
SFAKLEKEYNAKLGIYALDTGTNQTVAYHPNDRFAFASTSKSLAAGALLRQNSI
EALDERITYTREDLSNYNPITEKHVDTGMTLKELADASVRYSDSTAHNLILKKL
GGPSAFEKILRETGDTVTKSERFEPELNEVHPGETRDTSTPEAIAKTLQSFTLG
SALPTEKRELLVDWMKRNTTGDKLIRAGVPKGWEVADKTGAGSYGTRNDIAII
WPPNKKPIVLAILSNHDKEDAEYDDKLIADATKVVLDTLKVTNKN
- Amino acid sequence of *Clostridium Difficile* class A β-Lactamase SEQ ID NO. 39 (GenBank: HBH2479975.1): 
MKKLIFLIAIALVLSACNSNSSHAKELNDLEKKYNAHIGVYALDTKSGKEVKFNS
DKRFAYASTSKAINSAILLEQVPYNKLNKKIHINKDDIVAYSPILEKYVGKDITLKE
LIEASMAYSDNTANNKIIKEIGGIKKVKQRLKELGDKVTNPVRYEIELNYYSPKS
KKDTSTPAAFGKTLNKLIANGKLSKENKKFLLDLMLNNKSGDTLIKDGVSKDCK
VADKSGQAITYASRNDVAFVYPKGQSEPIVLVIFTNKDNKSDKPNDKLISETAK
SVMKEF
- Amino acid sequence of β -Lactamase class A from *Bacillus anthracis* SEQ ID NO. 40 (GenBank: PES83414.1):

MIVLNKFFNMLPYKKIVPVLFLSCATLIGCSNSNTQSEPPKQAKQENTVNHSFA
KLEKEFDAKLGIYALDTDTNQTVTYQSDERFAYASTHKALAVGVLLQKKSIEDL
NQRILYTREDLVNYNPITEKYVDTGMTLKELADASLRYSDNTAQNLILKQLGGP
SEFKKSLREIGDTVTNPERFEPELNEVHPGDTHDTSTPKALATSLQAFALGD
ILSTEKRNLLIDWMKRNTTGDKLIRAGVPGGWEVADKTGSGSYGTRNDIAIIWP
PNKKPIVLAILSNQGKEDAKYDDKLIAEATKIVLHALKTPNKQ

The 3D structure of the protein expressed by the Blamab gene of *Mycobacterium abscessus* (SEQ ID NO. 21) (Fig. 2b) was generated using the Alphafold artificial intelligence tool (Jumper, J., et al. 2021. Nature, 596( 7873), 583-589; Varadi, M., et al. 2022. Nucleic acids research , 50(D1), D439-D444).

The KPC-2 family proteins are the closest phylogenetically to Blamab (Brandt C, et al. 2017. Sci Rep. Feb 24; 7:43232.) and are also found in Gram negatives such as *Klebsiella pneumoniae,* or *Pseudomonas aeruginosa.* So much the KPC-2 protein of *Klebsiella pneumoniae* (Uniprot: Q9F663) as the KPC-2 protein from *Pseudomonas aeruginosa* consist of the amino acid sequence SEQ ID NO. 32:

MSLYRRLVLLSCLSWPLAGFSATALTNLVAEPFAKLEQDFGGSIGVYAMDTGSGATVS
YRAEERFPLCSSFKGFLAAAVLARSQQQAGLLDTPIRYGKNALVPWSPISEKYLTTGM
TVAELSAAAVQYSDNAAANLLLKELGGPAGLTAFMRSIGDTTFRLDRWELELNSAIPG
DARDTSSPRAVTESLQKLTLGSALAAPQRQQFVDWLKGNTTGNHRIRAAVPADWAV
GDKTGTCGVYGTANDYAVVWPTGRAPIVLAVYTRAPNKDDKHSEAVIAAAARLALEG

### LGVNGQ

More than 50% of people who die in hospital with acquired pneumonia die if their infection is caused by a strain that produces KPC-2. It was found that the 3D model of the protein encoded by the Blamab gene of *M. abscessus* contained the structural characteristics of the KPC-2 proteins (Fig. 2a), such as: the three helices, the Ω loop, the hinge region and the loop between helices α3 and α4 (Fig. 2b) (Galdadas I, et al., 2018. Sci Rep. Aug 27;8(1):12916). Furthermore, the BLASTp alignment of the sequences SEQ ID NO. 21 and SEQ ID NO. 32 shows a high level of conservation, especially in the residues essential for enzymatic activity: S70, E166, R164 and D179 (Fig.3).

In the design of the DNA construct for the expression of the protein encoded by the Blamab gene of *M. abscessus* to which a TEV protease recognition zone, a DYKDDDDK tag and a histidine tail were added in the c-terminal area. that allowed its purification (SEQ ID NO. 33 nucleotide sequence that encodes the protein encoded by the modified *M. abscessus Blamab gene*). The construct was flanked with BamHI restriction sites that allowed in-frame cloning into the pET26b expression vector. The optimization sequence for production in E. coli. K12 using EMBOSS Backtranseq (Madeira F, Pearce M, Tivey ARN, et al. 2022. Nucleic Acids Research Jul;50(W1):W276-W279).

Nucleotide sequence of synthetic construction for the expression of the fragment of the protein encoded by the modified *M. abscessus* Blamab gene, SEQ ID NO. 33:

ATGAAATACCTGCTGCCGACCGCTGCTGCTGGTCTGCTGCTCCTCGCTGCCCAG
CCGGCGATGGCCATGGATATCGGAATTAATTCGGATCCGGCGCCGGATGAACTG
GCGAGCCTGGAAAAAGATTTTGGCGGCCGCATTGGCGTGTATGCGCTGGATACC
GGCAGCGGCGATACCGTGGGCCATCGCGCGGATGAACGCTTTCTGATGTGCAGC
ACCGTGAAAACCTTTATTGTGAGCGCGATTCTGCGCCGCCTGAGCGAACCG
GGCCTGCTGGATCAGCGCATTCAGTATACCCAGAGCGATGTGCTGGAATGGGCG
CCGATTACCAGCCAGCATGTGAGCACCGGCATGACCGTGAGCGAACTGTGCGAT
GCGACCCTGCGCTATAGCGATAACACCGGCGCGAACCTGCTGATTACCCAGCTG
GGCGGCCCGAAAGAAACCGAAAAATTTGTGCGCAGCCTGGGCGATAACGTGACC
CGCATGGATCGCACCGAAGTGCAGCTGAACATTCCGGATGGCGATCTGGATACC
AGCACCCCGCAGCAGCTGGTGGCGAACCTGCGCCGCCTGGTGCTGGATGAAGG
CCTGGATAGCCGCGGCCGCGATCTGCTGACCGATTGGCTGAAACGCAACACCAC
CGGCGATCAGAGCATTCGCGCGGCGGTGCCGGCGGGCTGGACCGTGGCGGATA

AAACCGGCGGCGGCTTTAAAGGCGAAACCAACGATATTGCGGTGATTTGGCCGC
CGGGCCGCGCGCCGATTGTGATGGCGGTGCTGACCGTGCCGGAAGATCCGACC
AGCACCAAAGGCAAACCGACCATTGCGGCGGCGACCCGCATTGTGCTGCGCGCG
TTTGGCGCGGAAAACCTGTATTTTCAGAGCGATTATAAAGATGATGATGATAAAGA
GGATCCGAATTCGAGCTCCGTCGACAAGCTTGCGGCCGCACTCGAGCACCACCA
CCACCACCACTGA

The protein or polypeptide encoded by the nucleotide sequence SEQ ID NO. 33 consists of the amino acid sequence SEQ ID NO. 34, which comprises the amino acid sequence SEQ ID NO. 21. That is, SEQ ID NO. 34 comprises the class A β-lactamase fragment of *M.abscessus* ( SEQ ID NO. 21) that was used as an antigen to obtain antibodies (hereinafter antigenic protein). The protein with the amino acid sequence SEQ ID NO. 34 comprises the amino acid sequence SEQ ID NO. 21, and at its c-terminal end it comprises a c-terminal zone and a TEV protease recognition zone SEQ ID NO. 42: ENLYFQS, a DYKDDDDK tag (SEQ ID NO. 43) and a histidine tail HHHHHH (SEQ ID NO. 25) (underlined in SEQ ID NO. 34), for purification.

Amino acid sequence of the peptide comprising a fragment of the protein encoded by the Blamab gene of *M. abscessus* (*SEQ ID NO.* 21) + TEV protease recognition zone, a DYKDDDDK tag and an HHHHHH histidine tail, SEQ ID NO. 34:

MKYLLPTAAAGLLLLAAQPAMAMDIGINSDPAPDELASLEKDFGGRIGVYALDTGSGD
TVGHRADERFLMCSTVKTFIVSAILRRRLSEPGLLDQRIQYTQSDVLEWAPITSQHVST
GMTVSELCDATLRYSDNTGANLLITQLGGPKETEKFVRSLGDNVTRMDRTEVQLNIPD
GDLDTSTPQQLVANLRRLVLDEGLDSRGRDLLTDWLKRNTTGDQSIRAAVPAGWTVA
DKTGGGFKGETNDIAVIWPPGRAPIVMAVLTVPEDPTSTKGKPTIAAATRIVLRAFGAE
NLYFQSDYKDDDDKEDPNSSSVDKLAAALEHHHHHH

The synthetic DNA (SEQ ID NO. 33) was cloned into the pET26b vector for periplasmic expression in *E. coli.* Once the protein of SEQ ID NO. 34, the fragment corresponding to amino acids 1 to 30 of said sequence is immediately eliminated in the periplasm and is not part of the protein released into the periplasmic space that is subsequently used as an antigen in the process of obtaining antibodies. After cell lysis of induced *E. coli cultures,* the protein was purified by immobilized metal ion affinity chromatography. The concentration was calculated using Bradford, and the integrity was checked using denaturing PAGE gels and subsequent western blot analysis. Finally, nitrocefin assays were carried out to check the enzymatic activity of Blamab, which resulted in a Km = 45.3 ± 2.5 µM. The Km reflects the activity of the enzyme, which turned out to be in the range of other β-lactamases described in the literature.

### Example 2. Process for obtaining class A B-lactamase inhibitor antibodies.

Antibodies were obtained by serial selections from a "naïve" library of sdAbs that are displayed on the yeast surface. The library was obtained by assembly of synthetic sequences where the FRs are fixed and the CDRs contained increased variability through "error prone PCR". Since the sequences of the FRs were known and fixed, it was possible to determine the position and sequence of the CDRs. The FR1, FR2, FR3 and FR4 of the sdAbs of the synthesized library comprised the amino acid sequences SEQ ID NO. 44, SEQ ID NO. 45, SEQ ID NO. 46 and SEQ ID NO. 47, respectively.

Amino acid sequences of FR1, SEQ ID NO. 44:
QVQLQESGGGLVQAGGSLRLSCAAS

Amino acid sequence of FR2, SEQ ID NO. 45:
MGWYRQAPGKER

Amino acid sequence of FR3, SEQ ID NO. 46:
ADSVKGRFTISRDNAKNTVYLQMNSLKPEDTAVYYCA

Amino acid sequence of FR4, SEQ ID NO. 47:
YWGQGTQVTVSS

The original collection contained approximately 10^9 combinations of sdAbs that were generated by "error prone PCR". The conditions to introduce random mutations into the DNA were achieved by adding 2 µM to the PCR reaction. of 8-oxo-dGTP and 2 µM of dPTP. These two nucleotide analogues are introduced during the DNA extension step, and in a second conventional PCR they are replaced by natural nucleotides, consequently creating a new sequence. The new constructions were transformed into *S.cerevisiae cells* by electroporation. A library was generated with an approximate variability of 10^9 sdAbs. The library was amplified and stored at -80°C for later use.

For the selection process, the antigenic protein obtained in example 1 (which comprises the amino acid sequence SEQ ID NO. 21) was incubated with yeast cells that express the sdAbs library and subsequently stained with anti-His antibodies. and anti-DYKDDDDK bound to a fluorophore. This allows cells that express antibodies that recognize the antigenic protein to be trapped. The process must be repeated in at least four rounds of selection by MACS (Magnetic-activated cell sorting) and another round of selection by FACS (Fluorescence-activated Cell Sorting). This process is carried out as many times as necessary until it is observed an increase in the population of antigen-binding cells, a verification that is done using flow cytometry.

Once a collection containing about 200 antibodies was selected by FACS, ELISA tests were performed on all the variants in replicates. The method is similar to a sandwich ELISA, with the difference that it works with whole cells. The selected clones are grown in the FACS in several 96-well multiwell plates with a U-shaped bottom. Subsequently, the expression of the surface protein is induced and incubated with a concentration of 20 µg/mL of antigenic protein for 1 hour at 4°C. For immunological detection, anti - DYKDDDDK antibodies were used that recognize the antigenic protein and an anti-Rabbit conjugated to alkaline phosphatase was used as detection antibody. The 10 sdAbs with the highest antigen-binding capacity were selected and sequenced, cloned into the periplasmic expression vector pET26b for expression in *E.coli* and subsequently purified by chromatography. Of the 10 selected, 2 had identical sequences to others, and in one the protein could not be obtained, possibly due to its toxicity. Finally, 7 sdAbs were selected to study their affinity and inhibition capacity. The seven sdAbs obtained were named as B1, B2, B3, B4, B5, B6, B7, and presented the CDRs according to Table 1.

**Table 1. Single domain antibodies against β-lactamase.**

| Antibody Ref. | CDR1 | CDR2 | CDR3 | Antibody sequence |
|---|---|---|---|---|
| B1 | GNIFTVS S (SEQ ID | EFVAAISDGATT NY (SEQ ID NO. 23) | AYASRVGLTV (SEQ ID NO. 24) | SEQ ID NO. 29 |
| | NO. 22) | | | |
| B2 | GTIFVYP Y (SEQ ID NO.1) | EFVAGIGQGAT TYY (SEQ ID NO.2) | AYRYRDDVRRYGIDR YHT (SEQ ID NO.3) | SEQ ID NO. 16 |
| B3 | GTI FYYPY (SEQ ID NO.4) | EFVAGIDYGST TYY (SEQ ID NO.5) | VVYLVTRKGASQQD EIYS (SEQ ID NO.6) | SEQ ID NO. 17 |
| B4 | GTIFAQD Y (SEQ ID NO.7) | EFVASIAYGTIT YY (SEQ ID NO.8) | VYPTPVTDYI (SEQ ID NO.9) | SEQ ID NO. 18 |
| B5 | GNISDL GT (SEQ ID NO.10) | EFVAGIGTGSN TYY (SEQ ID NO.11) | VIAGRSYWIYFY (SEQ ID NO.12) | SEQ ID NO. 19 |
| B6 | GTISAVS G (SEQ ID NO.13) | EFVASINRGST TNY (SEQ ID NO.14) | ALHGTGRVHV (SEQ ID NO.15) | SEQ ID NO. 20 |
| B7 | GTISKAP V (SEQ ID NO.26) | EFVAGIASGSNT NY (SEQ ID NO.27) | AYVHI RRSG YIALT (SEQ ID NO.28) | SEQ ID NO. 30 |

Amino acid sequence of antibody B1, SEQ ID NO. 29:

MQVQLQESGGGLVQAGGSLRLSCAASGNIFTVSSMGWYRQAPGKEREFVAAISDGA
TTNYADSVKGRFTISRDNAKNTVYLQMNSLKPEDTAVYYCAAYASRVGLTVYWGQGT
QVTVSS

Amino acid sequence of the B7 antibody SEQ ID NO. 30:

MQVQLQESGGGLVQAGGSLRLSCAASGTISKAPVMGWYRQAPGKEREFVAGIASGS
NTNYADSVKGRFTISRDNAKNTVYLQMNSLKPEDTAVYYCAAYVHIRRSGYIALTYWG
QGTQVTVSS

### Example 3. Class A B-lactamase inhibitory antibody activity assays.

The β-lactamase inhibitory capacity was determined by nitrocefin colorimetric assays. This type of assay provides quantitative data that can be used for enzymological studies. The inhibition constant of antibodies B1 to B7 against β-lactamase comprising the sequence SEQ ID NO .21 was calculated.

10 µM β-lactamase was added with nitrocefin in a 100 µl reaction mixture volume at room temperature in a PBS solution, phosphate buffer saline pH 6.5. Nitrocefin hydrolysis was monitored using a microplate reader. The concentration of hydrolyzed nitrocefin (c) was calculated from its absorbance (A), molar extinction coefficient (ε) (20,500) and path length (l) values (0.5 cm) using Beer's law: A = εcl.

To evaluate the inhibition of nitrocefin hydrolysis in the presence of antibodies B1 to B7, the antibodies were added, in separate reaction mixtures at a final concentration of 10 µM, to a reaction mixture containing nitrocefin, establishing as a control the sample without antibodies.

To calculate Vmax and Km, the experiment was carried out with different concentrations of nitrocefin (40µM, 60µM, 80µM, 100µM, 200µM) and 1/v (initial velocity) was represented against 1/[c] (nitrocefin concentration) in a Lineweaver-Burk diagram. Once the Vmax and the Km were known, the Ki was cleared from the formula Ki = Km [I]/(Km, apparent - Km) where Km, apparent corresponds to the Km with inhibitor, and where "[I]" corresponds to the amount of antibody.

Thus, sdAbs B2-B6 presented inhibition constants (Ki) of between 2.4 and 5.2 µM calculated using the Lineweaver-Burk method, lower than those of B1 and B7 (in the case of B7 it does not present inhibition), which means that They are capable of inhibiting the enzymatic activity of β-lactamases at very low concentrations (Table 2).

**Table 2. Values of Vmax, Km and Ki obtained in the inhibition assays of antibodies B1-B7 against the β-lactamase of M.abscessus , with antibody and without antibody.**

| Antibody | Vmax (µM/min) | | Km (µM) | | Ki (µM) |
|---|---|---|---|---|---|
| | Control without antibody | With antibody | Control without antibody | With antibody | With antibody |
| B1 | 0.19 ± 0.003 | 0.26 ± 0.002 | 40±0.06 | 42.6 ± 0.9 | 15.6±0.3 |
| B2 | 0.22±0.01 | 0.25±0.008 | 45.3±2.5 | 64.4±2.2 | 2.4±0.08 |
| B3 | 0.29±0.01 | 0.34 ± 0.018 | 38.2±1.8 | 51.9 ± 2.8 | 2.7±0.15 |
| B4 | 0.29±0.01 | 0.28 ± 0.017 | 38.2±1.8 | 45.6 ± 2.7 | 5.2±0.31 |
| B5 | 0.22±0.01 | 0.23 ± 0.03 | 45.3±2.5 | 57.3±0.9 | 3.8 ± 0.06 |
| B6 | 0.19±0.01 | 0.26±0.025 | 44.3 ± 2.3 | 53.3±5.3 | 4.9 ± 0.49 |
| B7 | 0.19±0.01 | 0.18±0.03 | 44.3 ± 2.3 | 32.2±5.2 | N/A |

Furthermore, the affinity of antibodies B2, B5, B7 and a non-specific β-lactamases control antibody (antibody D1.1) was determined for the peptide comprising the amino acid sequence SEQ ID NO. 21 by ELISA assays. All antibodies were added at the same concentration. It was observed that antibodies B2, B5 and B7 presented a higher affinity than the D1.1 control in a comparative assay (table 3). A higher absorbance means that there is a greater amount of antibody bound. The B7 antibody has a higher affinity, but according to the data in Table 2, it does not produce inhibition, so the B7 antibody does not bind to the catalytic center.

**Table 3. Absorbance values at 405 nm of the ELISA affinity assay**

| Antibody | A405 |
|---|---|
| D1.1 | 0.029±0.0005 |
| B2 | 0.749 ± 0.167 |
| B5 | 0.798 ± 0.221 |
| B7 | 3.157± 0.118 |

Amino acid sequence of the control antibody D1.1, SEQ ID NO. 41:

QVQLQESGGGLVQAGGSLRLSCAASGTISYSYSMGWYRQAPGKDREFVATIAAGGS
TYYADSVKGRFTISRDNAKNTVYLQMNSLKPEDTAVYYCAVRYQPQPHPRVYHYYW
GQGTQVTVSS

### Example 4: Inhibition assays for β-lactamase activity of BlaMab, KPC-2, OXA-48, and VIM-2 by Inhibitory Antibodies.

An in vivo assay was conducted to test the inhibitory capacity of four β-lactamases from three resistant microorganisms: BlaMab from *Mycobacterium abscessus,* KPC-2 (UNIPROT Q9F663) and OXA-48 (UNIPROT Q6XEC0) from *Klebsiella pneumoniae,* and VIM-2 (UNIPROT B8QIQ9) from *Pseudomonas aeruginosa.* The assay utilized BL21(DE3) strain cells of Escherichia coli transformed with the expression vector pET26b(+) along with the gene sequence of each β-lactamase. Cells successfully transformed with the vector and insert were selected using a Kanamycin resistance gene (KanR) present in the vector. The presence and orientation of the insert were confirmed using PCR and sequencing. Additionally, the pET26b(+) vector has an IPTG-inducible promoter to activate the translation of the corresponding β-lactamase. The plasmid also includes a pelB export signal directing the β-lactamase to the periplasmic space of the bacteria for the inhibition assay with Ndis (nanobodies, single-domain antibodies).

To obtain Table 4 with the enzymatic constants (Km, Vmax, Kmi, and Ki) of BlaMab-2, KPC-2, VIM-2, and OXA-48 with B2 and B5, a quantitative assay was performed using a cephalosporin analog: nitrocefin. This compound changes color from yellow to red when hydrolyzed by β-lactamase, which can be quantified by measuring absorbance at 490nm. This method was used to study the membrane permeability of the antibodies in the bacteria as well as the inhibitory effect of B2 and B5 on the activity of BlaMab, KPC-2, OXA48, and VIM-2.

It was demonstrated that B2 and B5 are capable of penetrating the outer membrane and inhibiting both the β-lactamase of the Gram-positive bacterium M. abscessus, to which they are specific, and the β-lactamases of Gram-negative bacteria such as KPC-2 and OXA48 from Klebsiella pneumoniae, and VIM-2 from *Pseudomonas aeruginosa* (Table 4).

**Table 4: Enzymatic Constants (Km, Vmax, Kmi, and Ki) of BlaMab-2, KPC-2, VIM-2, and OXA-48 with B2 and B5 antibodies.**

| | Control without antibody | | **B2** | | |
|---|---|---|---|---|---|
| | Km(µM) | Vmax(µM/min) | Km(µM) | Vmax(µM/min) | Ki(µM) |
| BlaMab-2 | 9,34 ± 0,14 | 0,22 ± 0,0032 | 110,11 ± 2,068 | 1,15 ± 0,022 | 1,52 ± 0,028 |
| KPC-2 | 1,36 ± 0,11 | 0,098 ± 0,0077 | 120,91 ± 2,73 | 0,70 ± 0,016 | 0,060 ± 0,0014 |
| VIM-2 | 6,75 ± 0,15 | 0,013 ± 0,00029 | 39,61 ± 0,78 | 0,017 ± 0,00033 | 2,90 ± 0,057 |
| OXA-48 | 4,48 ± 0,062 | 0,013 ± 0,00018 | 6,62 ± 0,16 | 0,059 ± 0,0014 | 10,51 ± 0,25 |

| | Control without antibody | | **B5** | | |
|---|---|---|---|---|---|
| | Km(µM) | Vmax(µmM/min) | Km(µM) | Vmax(µM/min) | Ki(µM) |
| BlaMab-2 | 48,029 ± 1,28 | 0,26 ± 0,0069 | 159,53 ± 2,015 | 0,59 ± 0,007 | 5,90 ± 0,074 |
| KPC-2 | 1,36 ± 0,11 | 0,098 ± 0,0077 | 2,19 ± 3,62 | 0,50 ± 0,029 | 0,12 ± 0,0068 |
| VIM-2 | 4,35 ± 0,035 | 0,012 ± 9,58E-05 | 0,97 ± 0,026 | 0,0084 ± 0,00023 | 68,92 ± 1,85 |
| OXA-48 | 4,48 ± 0,062 | 0,013 ± 0,00018 | 2,261189±0,057958 | 0,061 ± 0,0016 | 30,77 ± 0,79 |

## Claims

1. An inhibitory antibody against class A β-lactamases from Gram-positive bacteria, or a fragment thereof, **characterized in that** said antibody or fragment thereof comprises an inhibition constant (Ki) of less than 10 µM against a β-lactamase comprising the amino acid sequence SEQ ID NO. 21.

2. Antibody or fragment thereof according to claim 1, wherein said antibody is from a human, mouse, rat, camelid, bird, a chimeric antibody or a humanized antibody.

3. Antibody or fragment thereof according to any one of claims 1 or 2, wherein said antibody is selected from the list consisting of IgY, IgG1, IgG2, IgG3 and HCAb.

4. Antibody or fragment thereof according to any one of claims 1 to 3, wherein said antibody is a monospecific or multispecific antibody.

5. Antibody or fragment thereof according to any one of claims 1 to 4, wherein the antibody is a monoclonal antibody.

6. Antibody or fragment thereof according to any one of claims 1 to 5, wherein the antibody fragment is selected from the list consisting of a single domain antibody (sdAb), an IgG1 heavy chain antibody (HCIgG1), a IgG2 heavy chain antibody (HCIgG2), an IgG3 heavy chain antibody (HCIgG3), an IgG4 heavy chain antibody (HCIgG4), a Fab fragment, an F(ab')2 fragment, an Fv fragment, Fv fragments linked by a disulfide bridge (sdFv), an Fd fragment and a scFv fragment.

7. Antibody or fragment thereof according to any one of claims 1 to 6, wherein the antibody is a single domain antibody.

8. Antibody or fragment thereof according to any one of claims 1 to 7, wherein said antibody or fragment comprises:
a) A CDR1 consisting of amino acid sequence SEQ ID NO. 1, a CDR2 consisting of amino acid sequence SEQ ID NO. 2, and a CDR3 consisting of amino acid sequence SEQ ID NO. 3;
b) A CDR1 consisting of amino acid sequence SEQ ID NO. 4, a CDR2 consisting of amino acid sequence SEQ ID NO. 5, and a CDR3 consisting of amino acid sequence SEQ ID NO. 6;
c) A CDR1 consisting of amino acid sequence SEQ ID NO. 7, a CDR2 consisting of amino acid sequence SEQ ID NO. 8, and a CDR3 consisting of amino acid sequence SEQ ID NO. 9;
d) A CDR1 consisting of amino acid sequence SEQ ID NO. 10, a CDR2 consisting of amino acid sequence SEQ ID NO. 11, and a CDR3 consisting of amino acid sequence SEQ ID NO. 12; or
e) A CDR1 consisting of amino acid sequence SEQ ID NO. 13, a CDR2 consisting of amino acid sequence SEQ ID NO. 14, and a CDR3 consisting of amino acid sequence SEQ ID NO. 15.

9. Antibody or fragment thereof according to any one of claims 1 to 8, wherein the antibody comprises the sequence SEQ ID NO. 16, SEQ ID NO. 17, SEQ ID NO. 18, SEQ ID NO. 19 or SEQ ID NO.20.

10. Antibody or fragment thereof according to any one of claims 1 to 9, wherein the antibody or fragment is linked to a therapeutic agent or a detectable marker.

11. Antibody or fragment thereof according to claim 10, wherein the therapeutic agent is an antibacterial agent.

12. Antibody or fragment thereof according to claim 10, wherein the detectable marker is selected from the list consisting of a fluorescent compound, a bioluminescent compound, a chemiluminescent compound, a radioisotope, a prosthetic group, a positron-emitting metal and a non-radioactive paramagnetic metal ion.

13. Antibody or fragment thereof according to any one of claims 1 to 12, wherein the antibody or fragment thereof is immobilized on a solid support, a lipid particle, a nanoparticle, on the surface of a virus or on the surface of a cell.

14. Pharmaceutical composition comprising the antibody or fragment according to any one of claims 1 to 13.

15. Pharmaceutical composition according to claim 14, wherein said composition comprises a pharmaceutically acceptable excipient and/or vehicle.

16. Antibody according to any one of claims 1 to 13, or pharmaceutical composition according to any one of claims 14 or 15, for use as a medicine.

17. Antibody or fragment according to any one of claims 1 to 13, or pharmaceutical composition according to any one of claims 14 or 15, for use in the treatment and/or prevention of an infection caused by a β-lactamase-producing bacteria in a subject.

18. Antibody, fragment or pharmaceutical composition for use according to claim 17, wherein the bacteria produces a class A β-lactamase.

19. Antibody, fragment or pharmaceutical composition for use according to any one of claims 17 or 18, wherein the bacteria is Gram positive.

20. Antibody, fragment or pharmaceutical composition for use according to any one of claims 17 to 19, wherein the bacteria is selected from the list consisting of *Mycobacterium abscessus, Pseudomonas aeruginosa, Mycobacterium tuberculosis, Mycobacterium leprae, Klebsiella pneumoniae, Listeria monocytogenes, Staphylococcus aureus, Enterococcus faecium, Enterococcus faecalis Streptococcus pneumoniae, Streptococcus agalactiae , Streptococcus pyogenes, Clostridium botulinum, Clostridium novyi, Clostridium septicum, Clostridium perfringens, Clostridium tetani, Clostridium difficile, Clostridium mangenotii, Bacillus anthracis, and Corynebacterium diphtheriae*

21. Antibody, fragment or pharmaceutical composition for use according to any one of claims 17 to 20, wherein the route of administration is selected from the list consisting of oral, intravenous, intramuscular, intramuscular, intraarterial, intravenous, vaginal, intradermal. subcutaneous, topical, ophthalmic and inhalation route.

22. Antibody, fragment or pharmaceutical composition for use according to any one of claims 17 to 21, wherein the infection is selected from the list consisting of pulmonary infection, urinary infection, skin infection, nervous system infection, bacteremia, septicemia, eye infection and sexually transmitted.

23. A polynucleotide encoding the antibody or fragment according to any one of claims 1 to 13.

24. An expression vector comprising the polynucleotide according to claim 23.

25. A host cell comprising the polynucleotide according to claim 23 or the expression vector according to claim 24.

26. A polypeptide consisting of the amino acid sequence SEQ ID NO. 21.

27. *In vitro* use of a polypeptide comprising an amino acid sequence with at least 80% identity with SEQ ID NO. 21, preferably consisting of the amino acid sequence SEQ ID NO. 21, for obtaining antibodies or fragments thereof.

28. Use according to claim 27, where the antibodies, or fragments thereof, are human, mouse, rat, camelid, bird, chimeric antibodies or humanized antibodies.

29. Use according to any one of claims 27 or 28, wherein the antibodies are selected from the list consisting of IgY, IgG1, IgG2, IgG3 IgG4 and HCAb.

30. Use according to any one of claims 27 to 29, wherein the antibodies are monoclonal.

31. Use according to any one of claims 27 to 30, wherein the antibody fragments are selected from the list consisting of single domain antibodies (sdAb), IgG1 heavy chain antibodies (HCIgG1), IgG2 heavy chain antibodies (HCIgG2 ), IgG3 heavy chain antibodies (HCIgG3), IgG4 heavy chain antibodies (HCIgG4), Fab fragments, F(ab')2 fragments, Fv fragments, disulfide-linked Fv fragments (sdFv), Fd fragments and scFv fragments .

32. Use according to any one of claims 27 to 31 wherein the antibodies are single domain antibodies.

33. Use according to any one of claims 27 to 32, wherein the antibodies are VHH single domain antibodies or humanized VHH single domain antibodies.

34. *In vitro* method for obtaining an antibody or antibody fragment against β-lactamase, where said method comprises:
a) providing a library of nucleic acids that encode antibodies or fragments thereof, wherein said antibodies or fragments comprise at least one immunoglobulin variable domain;
b) introducing the library from step a) into a surface display system selected from the list consisting of a phage surface display system, a prokaryotic surface display system and a eukaryotic surface display system;
c) contacting the exposure system of step b) with an antigen consisting of a polypeptide comprising an amino acid sequence with at least 80% identity with SEQ ID NO: 21, preferably consisting of the amino acid sequence SEQ ID NO. 21; and
d) select antibodies, or fragments thereof, that specifically bind to the antigen from step c).

35. Method according to claim 34, wherein the antibodies, or fragments thereof, are human, mouse, rat, camelid, bird, chimeric antibodies or humanized antibodies.

36. Method according to any one of claims 34 or 35, wherein the antibodies are selected from the list consisting of IgY, IgG1, IgG2, IgG3 IgG4 and HCAb.

37. Method according to any one of claims 34 to 36, wherein the antibodies are monoclonal.

38. Method according to any one of claims 34 to 37, wherein the antibody fragments are selected from the list consisting of single domain antibodies (sdAb), IgG1 heavy chain antibodies (HCIgG1), IgG2 heavy chain antibodies (HCIgG2 ), IgG3 heavy chain antibodies (HCIgG3), IgG4 heavy chain antibodies (HCIgG4), Fab fragments, F(ab')2 fragments, Fv fragments, disulfide-linked Fv fragments (sdFv), Fd fragments and scFv fragments.

39. Method according to any one of claims 34 to 38, wherein the antibodies are single domain antibodies.

40. Method according to any one of claims 34 to 39, wherein the antibodies are VHH single domain antibodies or humanized VHH single domain antibodies.
